# EUROPÄISCHE PATENTSCHRIFT

(11) **EP 1 139 968 B1**
(45) Veröffentlichungstag und Bekanntmachung des Hinweises auf die Patenterteilung: **09.03.2005**
(21) Anmeldenummer: 99967958.2
(22) Anmeldetag: 17.12.1999
(51) Int. Cl.: A61B 5/00

(54) **VORRICHTUNG ZUR EINKOPPLUNG VON ULTRASCHALLWELLEN IN EIN MEDIUM**
DEVICE FOR INJECTING ULTRASONIC WAVES INTO A MEDIUM
DISPOSITIF POUR INJECTER DES ONDES ULTRASONORES DANS UN MILIEU

(30) Priorität: 17.12.1998 DE 19861017
(43) Veröffentlichungstag der Anmeldung: 10.10.2001
(73) Patentinhaber: Fraunhofer-Gesellschaft zur Förderung der angewandten Forschung e.V., 80686 München (DE)
(72) Erfinder: GEBHARDT, Wolfgang, D-66583 Spiesen (DE); LICHT, Rudolf, D-66440 Blieskastel (DE)
(74) Vertreter: Rösler, Uwe, Dipl.-Phys.
(86) Internationale Anmeldenummer: PCT/EP1999/010094
(87) Internationale Veröffentlichungsnummer: WO 2000/035338

(56) Entgegenhaltungen:
- US-A- 4 378 699
- US-A- 4 944 186

## Beschreibung

### Technisches Gebiet

Die Erfindung bezieht sich auf eine Vorrichtung zur Einkopplung von Ultraschallwellen in ein Medium über eine Grenzfläche mit wenigstens einer Ultraschallwandlereinheit, die über ein Koppelmedium, das zwischen der Ultraschallwellen erzeugenden Einheit und der Grenzfläche vorgesehen ist, Ultraschallwellen in das Medium einkoppelt.

### Stand der Technik

Vorrichtungen der vorstehend genannten Gattung dienen der zerstörungsfreien Werkstoffuntersuchung und finden überdies in der Medizin weitverbreiteten Einsatz, um am menschlichen Körper Diagnosen im Körperinneren, wie bspw. bei der Schwangerenuntersuchung, durchführen zu können.

Die Wechselwirkung von Ultraschall vorzugsweise mit Festkörpern beruht ähnlich wie bei Licht in Glas, auf Absorption (Schwächung), Reflexion und Brechung. Reflexion und Brechung treten an der Grenzfläche zwischen zwei Stoffen mit verschiedenen physikalischen Eigenschaften auf, z.B. an einer Grenzfläche eines Körpers. Da diese Unterschiede insbesondere bei Verbundwerkstoffen oft gering sind, ist eine hohe Empfindlichkeit des Empfangsgerätes Voraussetzung, durch das die rückreflektierten Ultraschallwellen detektiert werden können. Oft sind Ultraschallsender und Ultraschallempfänger in einer Einheit integriert und als Ultraschallwandlersysteme bekannt. Um einen Ultraschallwandler als Sender und als Empfänger zugleich zu nutzen, werden in kurzen Zeitabständen Ultraschallimpulse ausgesandt und in den Pausen der reflektierte Ultraschall empfangen.

Aus der US 4,378,699 ist eine Vorrichtung gemäß dem Oberbegriff des Anspruchs 1, insbesondere ein Analysesystem zur Untersuchung von Proben mittels Ultraschall bekannt, bei dem die Probe in einer unter Überdruckbedingungen stehenden Kammer eingebracht ist, auf die zielgerichtet ein Ultraschallwellenbündel zu Analysezwecken gerichtet ist.

### Darstellung der Erfindung

Aufgabe der Erfindung ist es eine Vorrichtung zur Einkopplung von Ultraschallwellen in ein Medium über eine Grenzfläche mit wenigstens einer Ultraschallwandlereinheit, die über ein Koppelmedium, das zwischen der Ultraschallwellen erzeugenden Einheit und der Grenzfläche vorgesehen ist, Ultraschallwellen in das Medium einkoppelt, derart weiterzubilden, dass der Einkoppelwirkungsgrad, also das Maß mit der Ultraschallwellen in das Medium eingekuppelt wird, deutlich erhöht werden soll. Ebenso gilt es zum verbesserten Nachweis von den, am Medium reflektierten Ultraschallwellen eine innige Kopplung zwischen der Ultraschallwellen erzeugenden Einheit und dem zu untersuchenden Medium herzustellen.

Die Lösung der der Erfindung zugrundeliegenden Aufgabe ist im Anspruch 1 angegeben. Vorteilhafte Weiterbildungen sind Gegenstand der Unteransprüche sowie dem Beschreibungstext und den Ausführungsbeispielen zu entnehmen.

Im Falle einer luftgekoppelten Anregung von Ultraschallwellen, bei der Luft als Koppelmedium eingesetzt wird, wird zur entscheidenden Verbesserung der Energiebilanz erfindungsgemäß ein sogenannter Druckluftgleitschuh eingesetzt, eine Vorrichtung, die im Folgenden beschrieben wird.

Unter luftgekoppelter Anregung von Ultraschallwellen versteht man, daß die Ultraschallwandlereinheit Schallwellen in Luft erzeugt, die nach Durchlaufen einer mehr oder weniger langen Strecke unter einem geeigneten Winkel auf die Grenzfläche bzw. auf eine Festkörperoberfläche auftreffen und im Medium bspw. in einem Festkörper Raumwellen sogenannte Dichte- oder Scherwellen oder an der Festkörperoberfläche entlanglaufende Oberflächenwellen, sogenannte Rayleighwellen oder Kriechwellen, anregen. So können auch an plattenförmigen Materialien verschiedene Plattenwellenmoden angeregt werden.

Die erfindungsgemäße Vorrichtung gemäß dem Oberbegriff des Anspruchs 1, zeichnet sich dadurch aus, dass die zweite Öffnung der Grenzfläche unmittelbar zugewandt ist, durch die ein, aus dem Inneren des Volumens herrührender Gasstrom austritt.

Als Koppelmedium wird vorzugsweise Druckluft in das Innere des, von einem Gehäuse umgebenen, abgeschlossenen Volumens gegeben, die wiederum durch wenigstens eine Öffnung an der der Grenzfläche zugewandten Seite des Gehäuses ausströmt. Durch das gezielte Ausströmen der Druckluft an der Unterseite des Gehäuses des sogenannten Druckluftgleitschuhs, wird dieser im Wege des sogenannten hydrodynamischen Paradoxons an die Grenzfläche regelrecht angesaugt, wodurch sich eine innige Kopplung zwischen dem Gehäuse und der Grenzfläche ergibt. Dies wiederum führt zu einem weitgehend konstanten Abstand zwischen der Vorrichtung und der Grenzfläche, wodurch die Meßbedingungen erheblich verbessert werden.

Alternativ zum Effekt des hydrodynamischen Paradoxons kann die Intensität des Gasstromes weiter erhöht werden, sodaß sich eine Art Luftkissen zwischen der Vorrichtung und der Grenzfläche ausbildet, so daß die gesamte Vorrichtung in Art eines Hovercrafts über der Grenzfläche schwebt.

Der Hauptvorteil des druckluftbeaufschlagten, abgeschlossenen Volumens innerhalb des Druckluftgleitschuhs ist darin zu sehen, daß aufgrund der druckbedingten höheren Luftdichte innerhalb des Gehäuses Ultraschallwellen effektiver über die Grenzfläche in das Medium, das vorzugsweise als Festkörper ausgebildet ist, einkoppeln können. Typischerweise herrscht innerhalb des Druckluftgleitschuhs ein ca. 10 mal höherer Druck als in der Umgebung. Ultraschallwellen können somit 10 mal besser in das Medium eingekoppelt werden.

Die erfindungsgemäße Vorrichtung dient zur Ultraschallwelleneinkopplung, vorzugsweise an jenen technischen Oberflächen, die aufgrund von Reinheitsbedingungen oder ähnlichen Umständen nicht direkt mit einem Prüfkopf kontaktiert werden dürfen. Mit der erfindungsgemäßen Vorrichtung können über Grenzflächen höchst effektiv Ultraschallwellen eingekoppelt werden ohne dabei diese zu berühren und ohne großen technischen Aufwand hinsichtlich Dichtungsmaßnahmen, die zum Aufrechterhalten des Luftdruckes innerhalb des Gehäuses bzw. des Druckluftgleitschuhs verbunden sind, zumal sich die Druckbedingungen, aufgrund des vorstehend genannten hydrodynamischen Paradoxons von alleine einstellen.

Für alle weiteren Details der Erfindung wird die nachstehenden Ausführungsbeispiele verwiesen:

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnung exemplarisch beschrieben. Es zeigen:
- Fig. 1: Querschnitt durch eine vorteilhafte Ausführungsform,
- Fig. 2: Querschnitt durch eine vorteilhafte alternative Ausführungsform.

### Wege zur Ausführung der Erfindung, gewerbliche Verwendbarkeit

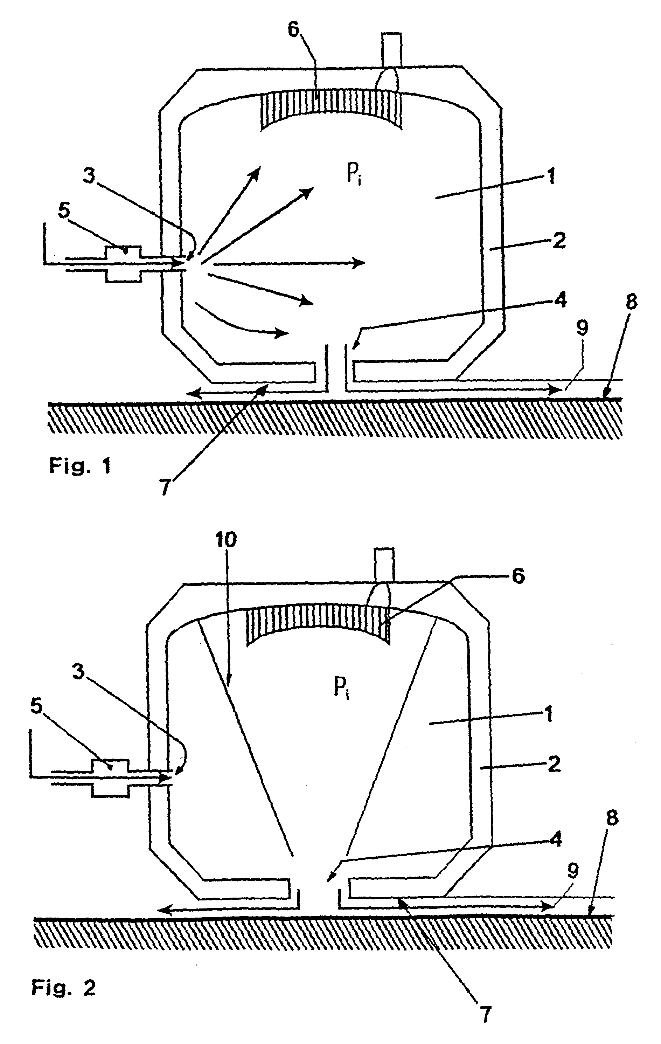

In ihrer einfachsten Ausführungsform (siehe Figur 1) strömt Druckluft in das abgeschlossene Volumen 1, das von einem Gehäuse 2 umgeben ist. Das Gehäuse 2 weist hierbei zwei Öffnungen 3, 4 auf. Durch die Öffnung 3 strömt Druckluft durch eine daran angeschlossene Druckluftleitung 5 in das Innere des Gehäuses 2. Durch die andere Öffnung 4 kann die Druckluft an Außen entweichen. Im Inneren des Gehäuses 2 ist eine Ultraschallwandlereinheit 6 vorgesehen, die vorzugsweise an der, der Öffnung 4 gegenüberliegenden Seite angebracht ist, so daß die Ultraschallwellen auf die Öffnung 4 gerichtet abgestrahlt werden können. Die Öffnung 4 dient in diesem Falle auch als Schallaustrittsöffnung.

Als Ultraschallwandlereinheiten können vorzugsweise zeitlich laufzeitgesteuerte Stapelwandler eingesetzt werden. Der Einbau von konventionellen Wandlern in den Druckluftgleitschuh ist ebenfalls möglich. Insbesondere für niederfrequente Anwendungen können die Einzelscheiben des Stapels auch phasengleich angeregt werden. Eingebaut werden können natürlich auch konventionelle Einzelschwingerwandler.

Die zwischen Unterseite 7 des Gehäuses und der Prüflingsoberfläche bzw. der Grenzfläche 8 sich ausbildende radiale Druckluftströmung 9 erzeugt zwischen beiden Flächen einen Unterdruck, der den Druckluftgleitschuh an die Prüflingsoberfläche 8 anzieht. Der sich verringernde Abstand zwischen Gleitschuhunterseite und Prüflingsoberfläche 8 erhöht weiter die Strömungsgeschwindigkeit, was seinerseits zu einer Erhöhung der Anpreßkraft führt. Gleichgewicht stellt sich ein, wenn sich die durch die Radialströmung erzeugte Anziehungskraft gleich der abstoßenden Kraft (verursacht durch den sich im Gleitschuhinnneren aufbauenden Druck) ist. Die Dicke des Luftspalts zwischen Gleitschuhunterseite und Prüflingsoberfläche sowie die Höhe des Drucks im Gleitschuhinneren hängen von der geometrischen Auslegung ab.

Die beschriebene einfachste Version des Druckluftgleitschuhs besitzt jedoch gewisse Nachteile, die durch die Turbulenzen des Gasstromes im Inneren des Gehäuses 2 bedingt sind: So kommt es insbesondere zu störenden Fluktuationen der Ultraschallimpulse bezüglich ihrer Gestalt und Amplitude im Inneren des Gehäuses 2. Um diese Störungen zu reduzieren, werden in das Innere des Gehäuses 2 geeignete Schallleitmittel 10 eingebaut, um die Ultraschallwellen entsprechend umzulenken und/oder zu konzentrieren bspw. auf die Schallaustrittsöffnung 4 zu konzentrieren. Insbesondere dienen die Schallleitmittel 10 einer räumlichen Abtrennung eines Raumbereiches, der von den Ultraschallwellen durchlaufen wird, von einem Raumbereich, in dem sich die, in das Innere des Gehäuses eingebrachte Gasströmung frei ausbilden kann. In Figur 2 ist als Schallleitmittel 10 ein Trichtereinsatz vorgesehen, der die von der Ultraschallwandiereinheit ausgehenden Ultraschallwellen in Richtung der Schallaustrittsöffnung 4 konzentriert.

Das Wechselwirkungsvolumen zwischen turbulenter Druckluft und Schall ist hiermit sehr stark eingeschränkt, was die Turbulenzeffekte entsprechend reduziert. Statt dem Trichter können grundsätzlich alle Einbauteile wie Leitbleche, Lochfilter usw. verwendet werden, die zu einer Laminarisierung oder Beruhigung der Luftströmung beitragen. Statt der Druckluft kann prinzipiell natürlich jedes andere Gas (z. B. CO₂) verwendet werden.

Desweiteren können Schallaustrittsöffnung und weitere Druckluftaustrittsöffnungen voneinander getrennt am Gehäuse angebracht werden.

Die Ultraschallwandlereinheit 6 kann je nach Anwendung senkrecht zur Prüflingsoberfläche 8 oder zur Erzeugung schräg einfallender Ultraschallwellen geneigt in das Gehäuse eingebaut werden. Werden Senden und Empfang mit zwei Ultraschallwandlern realisiert, so besteht die Möglichkeit, diese in getrennte Gleitschuhe oder in einen gemeinsamen Gleitschuh einzubauen. In letzterem Fall können beide Wandler getrennte Schallaustrittsöffnungen mit getrennten Einbauten zur Turbulenzunterdrückung oder gemeinsame Schallaustrittsöffnungen mit gemeinsamem Einbau zur Turbulenzunterdrückung besitzen. Die geometrische Anordnung (Schräglage, gegenseitiger Abstand) wird in Abhängigkeit von der Anwendung (Prüfung dicker Bauteile oder dünner Bauteile, Anregung von räumlichen Wellen, Oberflächenwellen oder Plattenwellen) angepaßt.

### Bezugszeichenliste

- 1: abgeschlossenes Volumen
- 2: Gehäuse
- 3,4: Öffnungen
- 5: Druckluftleitung
- 6: Ultraschallwandlereinheit
- 7: Unterseite
- 8: Grenzfläche, Prüflingsoberfläche
- 9: Druckluftströmung
- 10: Schallleitmittel

## Patentansprüche

1. Vorrichtung zur Einkopplung von Ultraschallwellen in ein Medium über eine Grenzfläche mit wenigstens einer Ultraschallwellen erzeugenden Ultraschallwandlereinheit (6), die über ein Koppelmedium, das zwischen der Ultraschallwandlereinheit (6) und der Grenzfläche (8) vorgesehen ist, Ultraschallwellen in das Medium einkoppelt, wobei die von der Ultraschallwandlereinheit (6) erzeugten Ultraschallwellen in ein abgeschlossenes Volumen (1) gerichtet sind, das wenigstens eine erste und eine zweite Öffnung (3, 4) aufweist, wobei durch die erste Öffnung (3) ein Gasstrom in das Innere des Volumens (1) gerichtet ist, der im Inneren des abgeschlossenen Volumens (1) für einen Überdruck sorgt und zugleich das Koppelmedium darstellt, und wobei durch die zweite Öffnung (4) ein aus dem Inneren des Volumens (1) herrührender Gasstrom (9) austritt, **dadurch gekennzeichnet, dass** die zweite Öffnung (4) der Grenzfläche (8) unmittelbar zugewandt ist.

2. Vorrichtung nach Anspruch 1,
**dadurch gekennzeichnet, dass** das abgeschlossene Volumen (1) von einem Gehäuse (2) begrenzt ist, in das die Ultraschallwandlereinheit (6) derart einsetzbar oder integriert ist, dass die Ultraschallwellen auf die, der Grenzfläche (8) unmittelbar zugewandten Öffnung (4) gerichtet sind.

3. Vorrichtung nach Anspruch 1 oder 2,
**dadurch gekennzeichnet, dass** der Gasstrom (9) aus Luft, vorzugsweise Druckluft besteht.

4. Vorrichtung nach einem der Ansprüche 1 bis 3,
**dadurch gekennzeichnet, dass** an der ersten Öffnung (3) eine Druckluftleitung (5) angeschlossen ist.

5. Vorrichtung nach einem der Ansprüche 2 bis 4,
**dadurch gekennzeichnet, dass** das Gehäuse (2) eine, der Grenzfläche (8) zugewandte Oberfläche (7) aufweist, in der wenigstens die zweite Öffnung (4) vorgesehen ist.

6. Vorrichtung nach Anspruch 5,
**dadurch gekennzeichnet, dass** das Gehäuse (2) in der der Grenzfläche (8) zugewandten Oberfläche (7) eine dritte Öffnung vorsieht, auf die die Ultraschallwellen durch entsprechende Ausrichtung der Ultraschallwandlereinheit (6) gerichtet sind.

7. Vorrichtung nach einem der Ansprüche 1 bis 6,
**dadurch gekennzeichnet, dass** im Inneren des abgeschlossenen Volumens (1) Schallleitmittel (10) zur Umlenkung und/oder Konzentration von Ultraschalwellen vorgesehen sind.

8. Vorrichtung nach Anspruch 7,
**dadurch gekennzeichnet, dass** die Schallleitmittel (10) Flächenteile, wie Leitbleche, sind, die einen ersten Raumbereich innerhalb des abgeschlossenen Volumens, in dem sich die Ultraschallwellen weitgehend ungestört von Gasströmungen ausbreiten können, von einem zweiten Raumbereich abgrenzen, in dem der Gasstrom geführt wird.

9. Vorrichtung nach Anspruch 7 oder 8,
**dadurch gekennzeichnet, dass** ein trichterförmig ausgebildetes Schallleitmittel (10) vorgesehen ist, das die Ultraschallwellen von der Ultraschallwandlereinheit (6) zur zweiten oder dritten Öffnung führt, so daß die Ultraschallwellen möglichst unbeeinträchtigt von der Gasströmung durch die zweite oder dritte Öffnung gelangen.

10. Vorrichtung nach einen der Ansprüche 5 bis 9,
**dadurch gekennzeichnet, dass** der, durch die, der Grenzfläche (8) zugewandte zweite oder dritte Öffnung (4) hindurchtretende Gasstrom (9) zwischen der, der Grenzfläche zugewandten Oberseite (7) des Gehäuses (2) und der Grenzfläche (8) radial zur zweiten oder dritten Öffnung (4) nach außen strömt, wobei ein Unterdruck entsteht, der das Gehäuse (2) soweit an die Grenzfläche (8) heranzieht, bis sich ein Gaskissen mit einer Dicke eingestellt, bei der sich durch den Unterdruck ausbildende anziehende Kräfte sowie durch den Massenimpuls des Gasstroms vorhandene abstoßende Kräfte zwischen dem Gehäuse (2) und der Grenzfläche im Gleichgewicht befinden.

11. Vorrichtung nach einem der Ansprüche 1 bis 10,
**dadurch gekennzeichnet, dass** die Ultraschallwandlereinheit (6) zwei Ultraschallwandler enthält, vorzugsweise ein Sendewandler und ein Empfangswandler.

12. Vorrichtung nach einem der Ansprüche 1 bis 11,
**dadurch gekennzeichnet, dass** das Medium (8), in das die Ultraschallwellen einkoppeln ein Festkörper ist.

13. Vorrichtung nach einem der Ansprüche 1 bis 12,
**dadurch gekennzeichnet, dass** das Medium (8), in das die Ultraschallwellen einkoppeln, biologisches Gewebe ist.

## Claims

1. A device for coupling in ultrasonic waves into a medium via a boundary surface (8), having at least one ultrasonic-waves-generating ultrasonic transducer unit (6) , which couples ultrasonic waves into said medium via a coupling medium provided between said ultrasonictransducer unit (6)and said boundary surface (8), with the ultrasonic waves generated by said ultrasonic transducer unit (6) being directed into a closed volume (1), which is provided with at least a first opening and a second opening (3,4), with a flow of gas, which ensures an overpressure inside said closed volume (1) and simultaneously represents said coupling medium, being directed into the interior of said volume (1) through said first opening (3), and with a flow of gas (9) coming from inside of said volume (1) exiting through said second opening (4),
wherein said second opening (4) directly faces said boundary surface (8).

2. The device according to claim 1,
wherein said closed volume (1) is bordered by a housing (2) in which said ultrasonic transducer unit(6) is insertable or integrated in such a manner that the ultrasonic waves are directed at said opening (4) directly facing said boundary surface (8).

3. The device according to claim 1 or 2,
wherein said flow of gas (9) is air, preferably compressed air.

4. The device according to one of the claims 1 to 3,
wherein a compressed-air line (5) is connected to said first opening (3).

5. The device according to one of the claims 2 to 4,
wherein said housing (2) has a surface (7), which is provided with at least said second opening (4), facing said boundary surface (8).

6. The device according to claim 5,
wherein said housing (2) in which said surface (7) facing said boundary surface (8) is provided with a third opening at which the ultrasonic waves are directed by corresponding alignment of said ultrasonic transducer unit (6).

7. The device according to one of the claims 1 to 6,
wherein sound-conducting means (10) for deflecting and/or concentrating ultrasonic waves are provided inside said closed volume (1).

8. The device according to claim 7,
wherein said sound-conducting means (10) are plane elements, such as baffle plates, which separate a first spatial zone inside said closed volume, in which ultrasonic waves can propagate for the most part without the interference by gas flows, and a second spatial zone in which said gas flow is directed.

9. The device according to claim 7 or 8,
wherein a funnel-shaped sound-conducting means (10) is provided which leads said ultrasonic waves from said ultrasonic transducer unit (6) to said second or third opening in such a manner that said ultrasonic waves pass through said second or third opening as unimpeded as possible by the gas flow.

10. The device according to one of the claims 5 to 9,
wherein said flow of gas (9) passing through said second or third opening (4) facing said boundary surface (8) flows between the upper side (7) of said housing (2) facing said boundary surface (8) and said boundary surface (8) radially in relation to said second or third opening(4) to the outside, with an vacuum developing which draws said housing (2) toward said boundary surface (8) to such a degree until a gas cushion is created having a thickness at which the forces of attraction being created by said vacuum and the forces of repulsion present due to the mass impulse of the flow of gas between said housing and said boundary surface are in equilibrium.

11. The device according to one of the claims 1 to 10,
wherein said ultrasonic-wave transducer unit (6) contains two ultrasonic transducers, preferably a transmitter transducer and a receiver transducer.

12. The device according to one of the claims 1 to 11,
wherein said medium (8) into which the ultrasonic waves couple in is a solid body.

13. The device according to one of the claims 1 to 12,
wherein said medium (8) into which the ultrasonic waves couple in is biological tissue.

## Revendications

1. Dispositif de couplage d'ondes ultrasonores dans un milieu sur une interface comportant au moins une unité de transduction d'ultrasons (6) productrice d'ondes ultrasonores, qui couple par l'intermédiaire d'un milieu de couplage, qui est prévu entre l'unité de transduction d'ultrasons (6) et l'interface (8), des ondes ultrasonores dans le milieu, dans lequel les ondes ultrasonores produites par l'unité de transduction d'ultrasons (6) sont dirigées dans un volume fermé (1), qui présente au moins une première et une deuxième ouverture (3,4), dans lequel un flux de gaz, qui assure une surpression à l'intérieur du volume fermé (1), est dirigé à l'intérieur du volume (1) à travers la première ouverture (3) et constitue en même temps le milieu de couplage et dans lequel un flux de gaz (9) brassé à l'intérieur du volume (1) ressort par la deuxième ouverture (4), **caractérisé en ce que** la deuxième ouverture (4) est directement tournée vers l'interface (8).

2. Dispositif selon la revendication 1,
**caractérisé en ce que** le volume fermé (1) est délimité par un boîtier (2), dans lequel l'unité de transduction d'ultrasons (6) est utilisée ou intégrée de telle sorte que les ondes ultrasonores soient dirigées vers l'ouverture (4) directement tournée vers l'interface (8).

3. Dispositif selon la revendication 1 ou 2,
**caractérisé en ce que** le flux d'air (9) est constitué d'air, de préférence d'air sous pression.

4. Dispositif selon une des revendications 1 à 3,
**caractérisé en ce que** une conduite d'air sous pression (5) est raccordée à la première ouverture (3).

5. Dispositif selon une des revendications 2 à 4,
**caractérisé en ce que** le boîtier (2) présente une surface supérieure (7) tournée vers l'interface (8), dans laquelle au moins la deuxième ouverture (4) est prévue.

6. Dispositif selon la revendication 5,
**caractérisé en ce que** le boîtier (2) possède une troisième ouverture dans la surface supérieure (7) tournée vers l'interface (8), vers laquelle les ondes ultrasonores sont dirigées en orientant en conséquence l'unité de transduction d'ultrasons (6).

7. Dispositif selon une des revendications 1 à 6,
**caractérisé en ce que**, à l'intérieur du volume fermé (1), des moyens conducteurs d'ultrasons (10) sont prévus pour dévier et/ou concentrer des ondes ultrasonores.

8. Dispositif selon la revendication 7,
**caractérisé en ce que** les moyens conducteurs d'ultrasons (10) correspondent à des pièces de surface, comme des déflecteurs, qui délimitent un premier espace à l'intérieur du volume fermé, dans lequel des ondes ultrasonores peuvent s'étendre essentiellement sans entrave à partir des écoulements de gaz, d'un deuxième espace, dans lequel le flux de gaz est introduit.

9. Dispositif selon la revendication 7 ou 8,
**caractérisé en ce que** un moyen conducteur d'ultrasons (10) réalisé en forme de trémie est prévu, lequel guide les ondes ultrasonores de l'unité de transduction d'ultrasons (6) vers la deuxième ou la troisième ouverture , de telle sorte que les ondes ultrasonores passent en subissant le moins de détériorations possible de l'écoulement de gaz au travers de la deuxième ou troisième ouverture.

10. Dispositif selon une des revendications 5 à 9,
**caractérisé en ce que** le flux de gaz (9) s'écoulant à travers la deuxième ou la troisième ouverture (4) tournée vers l'interface (8) s'écoule vers l'extérieur, radialement par rapport à la deuxième ou troisième ouverture (4) entre le côté supérieur (7) du boîtier (2), tourné vers l'interface et l'interface (8), moyennant quoi une dépression est générée, laquelle attire le boîtier (2) vers l'interface (8), jusqu'à ce qu'un gaz-coussin présentant une certaine épaisseur soit généré, dans lequel, sous l'effet des forces d'attraction formant la dépression ainsi que de l'impulsion de masse du flux de gaz, les forces répulsives présentes entre le boîtier (2) et l'interface se trouvent en équilibre.

11. Dispositif selon une des revendications 1 à 10,
**caractérisé en ce que** l'unité de transduction d'ultrasons (6) contient deux transducteurs d'ultrasons, de préférence un transducteur d'émission et un transducteur de réception.

12. Dispositif selon une des revendications 1 à 11,
**caractérisé en ce que** le milieu (8), dans lequel les ondes ultrasonores sont couplées, est un corps solide.

13. Dispositif selon une des revendications 1 à 12,
**caractérisé en ce que** le milieu (8), dans lequel les ondes ultrasonores sont couplées, est un tissu biologique.
